# EUROPEAN PATENT APPLICATION

(11) **EP 3 842 057 A1**
(43) Date of publication of application: **30.06.2021**
(21) Application number: 19797979.2
(22) Date of filing: 16.08.2019
(51) Int. Cl.: A61K 38/08, A61K 38/18, A61P 25/28

(54) **PHARMACEUTICAL COMBINATION COMPRISING EPIDERMAL GROWTH FACTOR AND SECRETAGOGUE PEPTIDE GHRP6 FOR THE RESTORATION OF BRAIN DAMAGE**

(30) Priority: 21.08.2018 CU 20180092
(71) Applicant: Centro De Ingenieria Genetica Y Biotecnologia, La Habana 11600 (CU)
(72) Inventor: GARCIA DEL BARCO HERRERA, Diana, Playa La Habana 11600 (CU); GUILLÉN NIETO, Gerardo Enrique, Playa La Habana 11600 (CU); VAZQUEZ CASTILLO, Mariela, Playa La Habana 11600 (CU); HERNÁNDEZ BERNAL, Francisco, Playa La Habana 11600 (CU); ZALDIVAR VAILLANT, Tatiana, Playa La Habana 11300 (CU); DÍAZ REYES, Pablo Arsenio, La Habana 10400 (CU); BALADRON CASTRILLO, Idania Caridad, La Habana 10400 (CU); BESADA PÉREZ, Vladimir Armando, Playa La Habana 11600 (CU); GONZALEZ BLANCO, Sonia, Playa La Habana 11600 (CU); BERLANGA ACOSTA, Jorge Amador, La Habana 17100 (CU); PÉREZ SAAD, Héctor Manuel, Playa La Habana 11600 (CU)
(74) Representative: V.O.
(86) International application number: PCT/CU2019/050006
(87) International publication number: WO 2020/038498

(57) **Abstract**

The invention discloses a pharmaceutical combination comprising epidermal growth factor (EGF) and the hexapeptide secretagogue GHRP6, useful for the restoration of brain damage, by stimulating mechanisms of differentiation and neuronal specialization in undifferentiated cells residing in the central nervous system or in neuronal cells. The invention also encompasses the use of the above mentioned biomolecules in the manufacture of a pharmaceutical combination for the restoration of brain damage. In addition, the invention provides a method of restoring brain damage in which a therapeutically effective amount of a pharmaceutical combination comprising EGF and GHRP6 is administered to a patient in need thereof. The efficacy of the pharmaceutical combination has a broad therapeutic window: it is independent of whether the pharmacological intervention occurs in the first or further hours after the brain damage, which broadens the therapeutic window.

## Description

### Technical field

The present invention relates to the field of medicine, in particular to the branch of neuropharmacology. Specifically, it is related to a pharmaceutical combination for the restoration of brain damage, through the enhancement and stimulation of mechanisms of differentiation and neuronal specialization in undifferentiated cells residing in the central nervous system, or in neuronal cells that acquire a phenotype and a function that allows them to replace other neurons that die due to ischemic damage or to proteinopathies. The therapeutic efficacy of the combination is independent of the moment of its application.

### Previous art

The pharmacological approach to tissue damage (ischemic, hemorrhagic, compression, deprivation of trophic factors or any other insult) in the central nervous system is a field that, although much studied, remains sterile in terms of effective therapeutic results, and quite unseccessful in its translation step clinical practice. This is due to the complexity and the very high specialization of the cellular components of the nervous system, which hinder the regeneration and replacement of the dead neurons and glial cells.

Most of the events that damage nervous system tissues have a worldwide incidence. For example, ischemic stroke is the second cause of death, and the third leading cause of disability worldwide (Krishnamurthi RV, Neuroepidemiology 2015; 45(3): 190-202). This happens not only to elder adults since young adults are also at risk of suffering ischemic strokes (Krishnamurthi RV, Neuroepidemiology 2015; 45(3): 177-89). In addition, traumatic accidents that affect the nervous system are very frequent, and cause disability with a high social cost (Greenwald BD, J Neurotrauma 2015; 32(23): 1883-92).

On the other hand, the number of diseases caused by morphological and functional alteration of intracellular proteins, known as proteinopathies are a serious health problem worldwide (Kawamata H, y otros,. J Cell Biol 2017; 216(12):3917-29), among them Alzheimer's disease, Parkinson's disease, Huntington's disease, and Amyotrophic Lateral Sclerosis. For the latter diseases, there is not yet a treatment aimed at brain restoration through the enhancement of neuronal plasticity, to reduce the progressive course of the disease and/or induce a significant recovery. Other causes of nervous system damage, such as trophic deficiencies (Lewis CM, Stem Cell Res Ther 2014; 5(2):32), are not as frequent as the above mentioned diseases and conditions but they represent a huge challenge for the scientific community, which has not been able, until now, to implement a therapy based on brain restoration, which be, significant and with impact on a better quality of life of these patients. Therefore, there is a need to find pharmacological interventions to promote brain restoration in contexts such as ischemic damage and proteinopathies, among others. The clinical assessment of recombinant proteins with neurotrophic activity, as candidate drugs for the treatment of many diseases of the nervous system, is a rational approach to induce brain restoration. However, the therapeutic benefit of said proteins in clinical practice has not been demonstrated so far (Henriques A, et al. Frontiers in Neuroscience. 2010; 4:32; Larpthaveesarp A, et al. Brain Sci 2015; 5(2): 165-77).

There is a perception, supported objectively by facts, that neurological disability is irreversible because none of the neuroprotective drugs that have been evaluated clinically until now, have managed to reverse the disability (Nicholson KA, Neurotherapeutics 2015; 12 (2):376-83). These drugs have not shown a positive impact on the quality-adjusted life years in survivors. Thus, finding therapeutic alternatives, that have an impact on the reduction of disability related to nervous system disorders, such as those caused by ischemic damage, by proteinopathies, or other causes of permanent neuronal damage is currently crucial. Said therapeutic alternatives should promote brain restoration in patients suffering from central nervous system damage.

Another criterion is that the therapeutic approach to ischemic brain damage with a neuroprotective approach, by thrombolysis (Mowla A, et al, J Neurol Sci 2017; 376:102-5) or by non-thrombolytic treatment (Matsuo R, et al. Stroke 2017; 48(11):3049-56), must be carried out before elapsed a period of 4 hours, since the onset of symptoms. This constitutes one of the major limitations of the neuroprotective treatments known to date.

It is important to differentiate the fact that the neuroprotective therapeutic approaches are directed towards the inhibition of primary neurodegenerative events. In contrast, therapeutic approaches for brain restoration are directed towards the stimulation of compensatory regenerative mechanisms (Francardo V, et al., Exp Neurol 2017; 298 (Pt B): 137-47).

Patent document WO2002/053167 describes a combination of the Epidermal Growth Factor (EGF) and the hexapeptide secretagogue known as GHRP6, for its acronym in English (*Growth Hormone Releasing Hexapeptide*). The components of the combination can be used, simultaneously or sequentially, in the prophylaxis of cell damage due to arterial blood deficit. Although it mentions the use of this combination for regenerative or trophic purposes, its use or of its separate components for brain restoration is neither demonstrated nor suggested. On the other hand, WO 2006/092106 describes the use of the EGF-GHRP6 combination for the treatment of autoimmune disorders of the nervous system. However, said patent document neither demonstrates nor suggests the ability of the combination to restore brain damage. In this invention the amounts of EGF with therapeutic efficacy are in the range of 1-10 µg / kg of body weight.

Additionally, *in vivo* investigations have evidenced that the EGF-GHRP6 combination has a neuroprotective effect in experimental models of global and focal brain infarction (Garcia DB-H., Et al. Restor Neurol Neurosci 2013; 31(2):213-23). In these models, the combination reduces the number or the severity of neurological symptoms, decreases the infarct volume, preserves neuronal density, and increases survival in the infarcted animals, as compared to vehicle treated control animals. This neuroprotective effect only occurs if treatment is initiated within 4 hours after the ischemic insult. In addition, both components of the combination are administered in the order of the hundreds of micrograms of active ingredient per kilogram of weight (Subiros N, et al. Neurol Res 2015; 38(3): 187-195). These studies in animal models showed therapeutic efficacy of the pharmacological intervention only in a very short period of time, which was similar to the therapeutic window described for other neuroprotective interventions (Alberts MJ. Circulation 2017; 135(2): 140-2) with dosages in the order of 100 µg of EGF per kg of body weight and 600 µg of GHRP6 per kg of body weight. Previous studies did not demonstrate therapeutic efficacy in terms of brain restoration. Also, when the administration of the pharmacological combination occurs 4 hours after the insult, there are no indications of therapeutic efficacy, neither clinically, nor histologically in terms of volume reduction of brain infarction. On the other hand, the use of the EGF-GHRP6 combination in neurological recovery has also been studied in an animal model of axonal pathology, which mimics motor neuron diseases (Del Barco DG et al., Neurotox Res 2011; 19(1): 195-209). A therapeutic effect was reached with dosages in the order of 200 µg per kilogram of weight for EGF, and of 660 µg per kilogram of weight for GHRP6. Neuronal plasticity is described in literature as the ability of the central nervous system to adapt in response to changes in the environment or due to insults. Moreover, neural plasticity is the capacity of neuronal groups to respond functionally, and neurologically, to deficiencies secondary to insults, or assuming the role of other injured neuronal groups, based on a synaptic reorganization and on the possibility of growth of new synapses from a neuron or from several damaged neurons (Kaas JH. Neural Plasticity A2 - Smelser, Neil J. En: Baltes PB, editor. International Encyclopedia of the Social & Behavioral Sciences. Oxford: Pergamon; 2001. p. 10542-6).

There is an endogenous physiological response of neuronal plasticity, which is not enough to supply the neuronal and glial cells to replace the lost complex functions. So far, neuronal plasticity to promote brain restoration has been tried and achieved through methods that include physical or cognitive training, but in no case has a pharmacological intervention been successfully used for this purpose. Therefore, there is a need for pharmacological interventions to promote brain restoration. It is desired that these therapeutic alternatives have an impact on reducing disability resulting from conditions of the nervous system, such as those caused by ischemic damage, proteinopathies, or other causes of permanent neuronal damage.

### Explanation of the invention

This invention contributes to solve the above mentioned problem by providing a pharmaceutical combination for the restoration of brain damage comprising the EGF and the growth hormone secretagogue peptide GHRP6. Said combination is effective in the enhancement of neuronal plasticity in a context of previous insult, such as ischemic damage, proteinopathies, or other causes of permanent neuronal damage, exception for autoimmune diseases.

In the context of the present invention, the term "pharmaceutical combination" refers to a combination of two active pharmaceutical ingredients, with various mechanisms of biological action, some common and others exclusive, where such ingredients or active substances are administered simultaneously or sequentially in the course of treatment to a patient in need.

The growth hormone secretagogue peptide GHRP6 is well known to those versed in this field of art. It has the following amino acid sequence: His-D-Trp-Ala-Trp-D-Phe-Lys-NH₂. EGF is a growth factor discovered in the last century, which is applied in the pharmaceutical field, in cosmetics, and in tissue engineering.

The "restoration of brain damage", in the context of the invention, requires the regeneration of the neuronal and glial components, supported by the activation of neuronal plasticity mechanisms. The routes for the activation of such mechanisms are induced when, for example, the expression of the gene encoding galectin-3 is suppressed, simultaneously with the increase in the expression of the gene encoding hemopexin, whose expression can be measured by nanoLC-MS (liquid chromatography of nanofluids coupled to mass spectrometry), as described by Martinez and others (Biochemistry and Biophysics Reports 5 (2016) 379-387). Galectin-3 is a lectin that contributes to the activation of microglia and, therefore, helps to expand and extend inflammation in the brain, which hinders endogenous or induced regeneration. It has been associated with a worse prognosis in patients (Denes, A., y otros, Brain Behav. Immun. 2010; 24: 708-723). Hemopexin, on the other hand, is a protein whose neuroregenerative function is associated with the stimulation of angiogenesis after damage by brain ischaemia and/or reperfusion.

### (Dong B y otros, BMC Anesthesiol 2018; 18:2)

In the present invention, the term "neuronal plasticity", also known as "neuroplasticity", is described as the ability to establish new neuronal circuits (by other neurons or cells that differentiate towards a neuronal phenotype), which allow the performance of complex nervous functions that were lost or impaired, due to ischemic, toxic, surgical, traumatic, proteinpathies, genetic disorders or other causes of permanent neuronal damage, except for autoimmune diseases. This potentiation contributes to brain restoration.

In an embodiment of the invention, the pharmaceutical combination comprises between 0.3 µg and 0.9 µg of EGF per kg of body weight, and between 30 µg and 80 µg of GHRP6 per kg of body weight, this components exhibit synergic effect. The coadministration of these active ingredients, in a specific dosage regime and period of time, showed a therapeutic efficacy superior to that described in the literature preceding this invention. The brain restoration achieved with the abovementioned pharmaceutical combination is evidenced by the preservation of complex functions, such as memory and learning capacity, and by an undoubted and rapid recovery of the motor, sensory and cognitive capacities of the treated patients. Surprisingly, the results showed that in very adverse scenarios of neurological damage, with more than 80% of injured motor neurons, as occurs in Amyotrophic Lateral Sclerosis, brain and spinal restoration is possible by stimulating and potentiating the physiological neuronal plasticity and, therefore, it is possible to reverse the characteristic torpid clinical course of this disease.

Said pharmaceutical combination favors, to some extent, the substitution of the lost tropism as a consequence of the primary damage. Advantageously, this enhancement of the neuronal plasticity mechanisms translates into therapeutic efficacy, without being restricted to the pharmacological intervention performed in the first hours after brain damage. This is a remarkable difference with the neuroprotective interventions of previous art. Furthermore, this enhancement of the neuronal plasticity mechanisms is decisive in brain restoration because the physiological response of neuroplasticity triggered immediately after an insult, is not enough for the replacement of the lost tropism as a result of primary damage, and do not have a significant impact on the neurological recovery of the patient in need . The pharmaceutical combination of the present invention promotes brain restoration in diseases of the nervous system that present a motor, sensory, autonomic and cognitive disabilities which are caused by ischemic, toxic, surgical, traumatic, by proteinopathies, genetic disorders or other causes of permanent neuronal damage, which do not include autoimmune diseases.

In one embodiment of the invention, the components of the EGH-GHRP6 combination are parenterally administered either sequentially or simultaneously. Within the routes for parenteral administration of the components of the combination are the intravenous, intrathecal, intracerebroventricular, intra-cistern magna or intranasal routes. In particular, without limiting it, intrathecal administration is performed by lumbar puncture and/or the use of a device designed for that kind of administration. For the intranasal route, the administration of the combination is aimed to reach the prolongations of the olfactory bulbs. In one embodiment of the invention, the combination consists of a kit comprising: a) a container or vial containing EGF and pharmaceutically acceptable excipients or vehicles, and b) a container or vial containing GHRP6 and pharmaceutically acceptable excipients or vehicles. In a particular embodiment, in the kit, the EGF is in the range of 5 µg to 80 µg per container, and the GHRP6 is in the range of 0.5 mg to 6 mg per container.

In another aspect, the invention contemplates the use of EGF and GHRP6 for the manufacture of a pharmaceutical combination for the restoration of brain damage. The use of this pharmaceutical combination for brain restoration is particularly advantageous. The synergy between both components stimulates the differentiation of stem cells or undifferentiated neurons, or G0 stage cells, to become cells with a neuronal and/or glial phenotype, with potential to acquire the functions of the neurons and glia that were lost, as a result of a previous insult of different nature. The use of this pharmaceutical combination, preferably in the dosages comprised in the present invention, has particular advantages because it is not associated with moderate or severe adverse effects. This therapeutic modality does not represent any risk for the patients.

In an embodiment of the invention, the combination is used in the restoration of brain damage after brain infarction. Surprisingly, this pharmaceutical combination produces a reversal of motor disability resulting from brain infarction, both hemorrhagic and ischemic. The synergism that exists between the components of the pharmaceutical combination of the invention translates into brain restoration, which is objectively expressed in a reduction of disability, in a substantial improvement in the quality of life and in the survival of the treated patients. These efficacy results were independent of the time of the first administration, that is, they were not associated with a narrow therapeutic window. This breaks the existing paradigm in relation to neuroprotective interventions. In addition, it is unexpected, in light of the results obtained with a combination of EGF and GHRP6 in animal models of brain ischemia, and that described for other similar clinical interventions (Mowla A, et al., J Neurol Sci 2017; 376:102-5), where therapeutic efficacy is demonstrated only when the treatment begins before 4 hours of the ischemic insult.

On the contrary, the administration of the pharmaceutical combination of the invention is not limited to the characteristic therapeutic window, of approximately between 4 to 6 hours (for neuroprotective, thrombolytic or non-thrombolytic interventions), in the case of ischemic disorders. Therefore, in a clinical essay of the invention, the first application of the combination took place between the initial moment of diagnosis of brain infarction and 24 hours after diagnosis.

Additionally, the pharmaceutical combination of the invention was used, compasionally, in newborns severely affected by severe neonatal hypoxia. Very favorable result was obtained, not only in the survival of these neonates, but also in the brain restoration through the enhancement of the neuronal plasticity. The evaluation at the end of the first year of life of those infants showed a lower than expected number of neurological sequelae considering the magnitude and severity of the neonatal hypoxia. Similarly and surprisingly, it was shown that the pharmaceutical combination of the present invention improves muscle strength and the swallowing reflex and reduces the progression of dysarthria in compassionately treated patients with Amyotrophic Lateral Sclerosis, who presented bulbar onset.

Therefore, the invention also contemplates a method of restoring brain damage, wherein a therapeutically effective amount of a pharmaceutical combination comprising EGF and GHRP6 is administered to patients in need thereof. In an embodiment of the invention, the therapeutically effective amount of EGF is between 0.3 µg and 0.9 µg of this growth factor per kg of body weight, and the therapeutically effective amount of GHRP6 is between 30 µg and 80 µg of the GHRP6 per kg of body weight of the patient. This approach of the invention is applicable when the damage is of an ischemic, toxic, surgical, traumatic nature, by proteinopathies, genetic disorders, or by other causes of permanent neuronal damage except for autoimmune diseases.

In a particular embodiment, the method of the invention is applied when the brain damage is caused by brain infarction or severe brain hypoxia of the newborn. In another embodiment, the method of the invention is applied in the context of extensive and chronic damage of motor neurons, as is the case of Amyotrophic Lateral Sclerosis, because it reduces the rate of progression of said disease, and also reduces the severity of the characteristic neurological symptoms. The combination is administered in the treatment of motor neuron diseases of progressive and torpid course, to improve muscle strength, swallowing reflex and to reduce dysarthria.

The therapeutic combination that is administered in the brain restoration method of the present invention, in addition to enhancing neuronal plasticity, constitutes in itself a trophic support similar to that which is generated physiologically during the embryonic and fetal stages. This not only favors the neurons and/or glial cells, but also all the components of the neurovascular unit. Therefore, the trophic support represented by said pharmaceutical combination mimics the physiological context of the tissues of the nervous system.

### Brief description of the figures

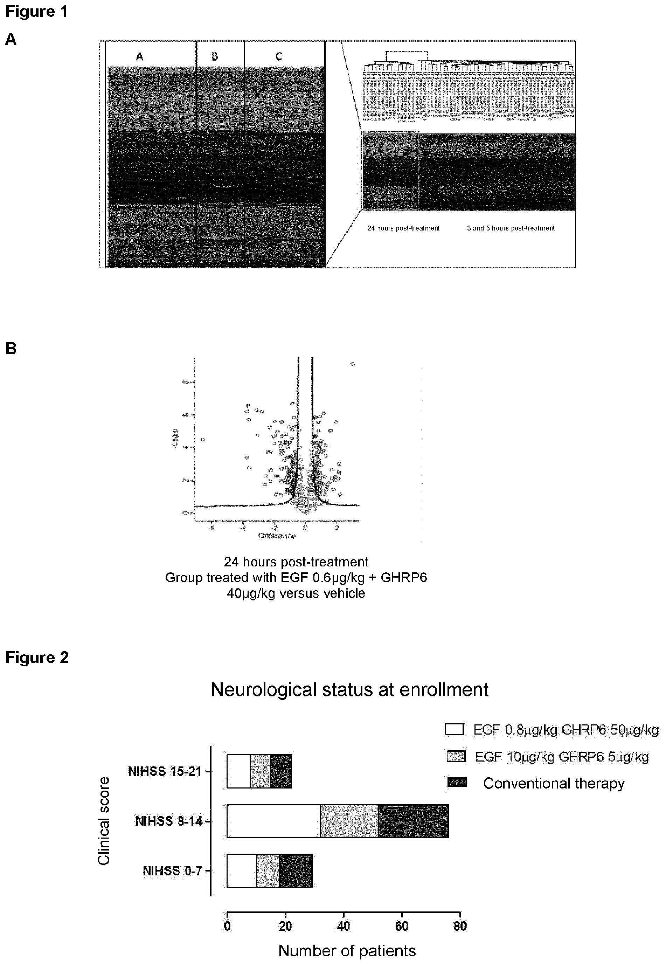

**Figure 1****.** Graphical representation of results found in proteomics experiments. Figure 1A represents the heat map of all proteins identified in each chromatographic cycle, demonstrating a different expression profile 24 hours after treatment of the rats with 0.6 µg of EGF/kg of body weight and 40 µg of GHRP6 / kg body weight (A), with respect to vehicle treatment (C), in rats with focal brain infarction, and also with respect to the sham operated control group (B). Figure 1B represents the volcano graphs, showing the differentially expressed proteins in the experimental group treated with the combination (0.6 µg of EGF/kg of body weight and 40 µg of GHRP6/kg of body weight), with respect to the control group treated with vehicle.
**Figure 2****.** Stratification of patients with brain infarction, according to clinical neurological severity at the time of diagnosis. Patients were randomly assigned to three treatment groups. Neurological clinical severity was assessed according to the NIHSS scale criteria.

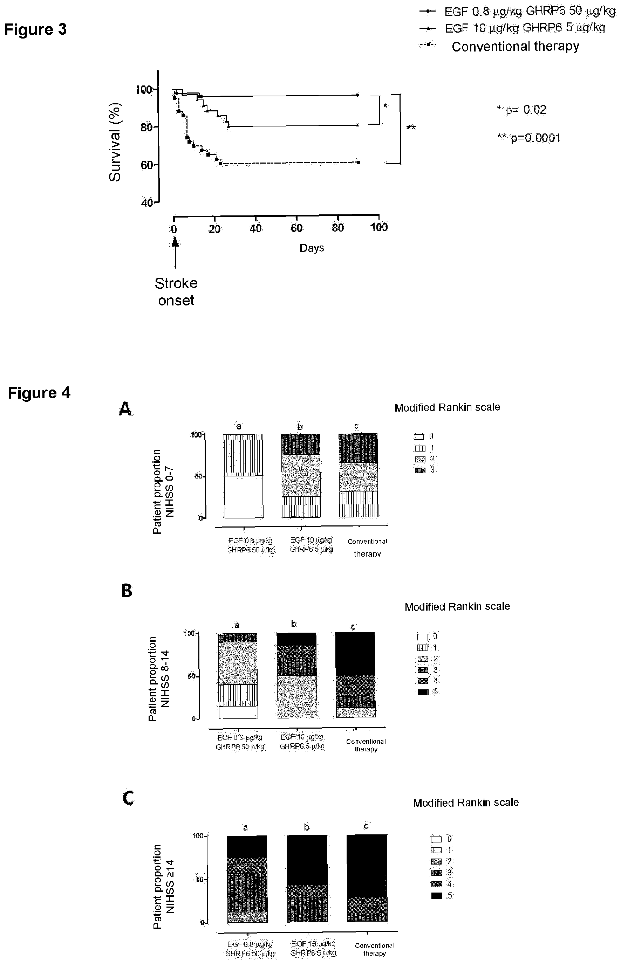

**Figure 3****.** Survival percentage of patients with brain infarction treated with two dose levels of the combination EGF and GHRP6, or with conventional therapy.
**Figure 4****.** Distribution of the results observed in the patients, according to the neurological evaluation based on the modified scale of Rankin, at 90 days after the onset of the brain infarct symptoms. Patients were treated with two dose levels of the EGF-GHRP6 combination, or with conventional therapy. The A-C panels correspond to the severity of the brain infarction, evaluated by the NIHSS scale at the time of diagnosis. Different letters indicate significant differences, p <0.001, in all cases, according to Chi square for proportion comparison test.

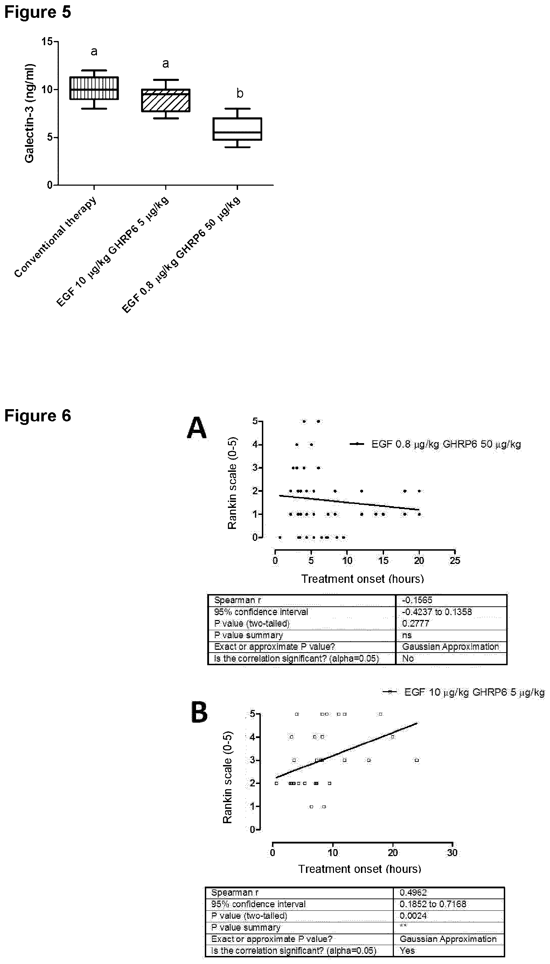

**Figure 5****.** Comparison of serum levels of galectin-3 in patients treated with conventional therapy, or with two dose levels of the EGF-GHRP6 combination, through the use of box and whisker diagrams. Differences between the three groups were compared using the Kruskal Wallis test, followed by the Dunn test. Equal letters indicate that there are no significant differences, different letters indicate statistically significant differences.
**Figure 6****.** Linear regression analysis demonstrating that in the group of patients treated with the pharmaceutical combination of 0.8 µg of EGF/kg of body weigh and 50 µg GHRP6/kg of body weigh there is no significant correlation between the starting time of the treatment and the clinical evolution in 90 days, according to the Rankin scale (0-5) **(A).** In the group that receives the combination of 10 µg of EGF/kg of body weight and 5 µg of GHRP6/kg of body weight, a positive and significant correlation is demonstrated between the time of initiation of treatment and clinical evolution according to the Rankin scale (0-5) **(B).**

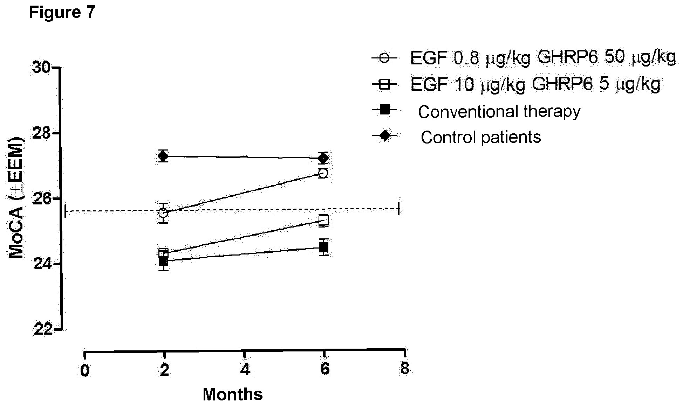

**Figure 7****.** Analysis of cognitive assessment, according to the Montreal scale (MoCA) at 2 and 6 months after brain infarction, in patients treated with combination therapy, at two dose levels of the EGF-GHRP6 combination, or with conventional therapy. It also includes a control group of 30 patients affected by non-neurological diseases. The asterisks indicate statistically significant differences, according to the Kruskal Wallis test, followed by the Dunn test.

### Detailed description of embodiments / examples of embodiments

### Example 1. Analysis of the differential expression of proteins that induces the treatment with EGF and GHRP6, in the zone of ischemic penumbra in rats with focal brain infarction.

In order to know the effect of the treatment with the of EGF-GHRP6 combination in the penumbra ischemic area, an experiment was carried out in rats with focal brain infarction, induced by intrabrain injection of endothelin 1. Two hours after intrabrain endothelin 1 injection rats were treated with the EGF-GHRP6 combination (0.6 µg/kg body weight of EGF and 40 µg/kg body weight GHRP6, n = 9) or with the vehicle (n = 10). Both experimental groups were followed and animals of each group were sacrificed, together with the animals of the sham operated group, at 3, 5 and 24 hours after the treatments with the referred pharmaceutical combination. Brain issue homogenates of all animals were prepared and pooled into three groups. The following procedures were performed: total proteins, enzymatic digestion, analysis of the peptide mixture by Liquid Chromatography/Mass Spectrometry (LC-MS/MS), and protein identification. The latter was carried out in sequence databases based on the MS/MS spectra, reducing the search to the Rattus norvegicus taxonomy of the Swissprot database (29 982 proteins, April 2016). For free labeling quantification, the Perseus program v.1.5.2.6 was used as a statistical tool. Not less than three replicates quantified for each protein were considered. A paired test was performed for each condition of interest, in relation to sham control animals, or between the EGF- GHRP6 combination treated and untreated animals. The null hypothesis was rejected for those proteins with a change factor ≥ 1.5 and a p-value less than 0.05. For the group of proteins with significant changes, a FDR (false discovery rate) of 5% was also considered.

The quantitative analysis of each ischemic animal sample was performed by comparison with the sham operated animals at the same time. Additionally, animals with focal brain infarction treated with the EGF-GHRP6 combination were compared with those treated with vehicle. The greatest amount of proteins that change their expression level was identified by the action of the combination at 24 hours after treatment. 97 proteins increased their expression levels and 124 proteins decreased. The results found in the proteomics experiments can be seen in the Figure 1. Among the proteins that significantly decreased their expression levels in the ischemic penumbra zone, due to the effect of the combined EGF- GHRP6 treatment are galectin-3 and the exitatory amino acid transporter type 2. On the contrary, the proteins hemopexine, neudesin, cytoglobin, parvalbumin and calbindin significantly increased their expression levels.

### Example 2. Effect of the administration of the EGF-GHRP6 combination in brain restoration in patients surviving brain infarction.

Fifty patients who suffered brain infarction of ischemic etiology were treated with the EGF-GHRP6 combination from the time they were diagnosed. The diagnosis included a time interval of up to 24 hours after the onset of symptoms. The treatment was carried out for a week, and consisted of the parenteral sequentially administration of 0.8 µg/kg body weight of EGF, and of 50 µg/kg body weight of GHRP6, every 12 hours. A second group of 35 patients was treated with the EGF-GHRP6 combination: 10 µg/kg body weight of EGF and 5 µg/kg body weight of GHRP6, following the same administration scheme. Another group of 42 patients with brain infarction, of ischemic etiology, was treated with the conventional therapy, which basically consisted in treating symptoms and complications.

All the patients were evaluated and scored according to the brain infarction scale of the National Institute of Health of the United States (NIHSS), which reflected their neurological status at the time of diagnosis. This evaluation allowed stratification of patients into three groups: mild brain infarction (NIHSS 0-7), moderate brain infarction (NIHSS 8-14), and severe brain infarction (NIHSS greater than 14). This neurological status of the patients at the time of diagnosis, starting point, by study group, is shown in Figure 2, where it can be seen that there were no differences in the mean neurological score of each group. Subsequently at day 90, the inicial NIHSS score was the reference (as starting point) to compare with the neurological status resulting from the treatments, evaluated by the modified Rankin scale. Survival analysis showed that 96% of patients survived in the group treated with the EGF-GHRP6 combination of 0.8 µg/kg body weight of EGF, and of 50 µg/kg body weight of GHRP6 and 60% of patients survived in the group that received the conventional therapy (p = 0.0001, according to the Manx Cox test of the logarithmic ranges). As compared to the group treated with the other pharmaceutical combination (10 µg/kg body weight of EGF, and of 5 µg/kg body weight of GHRP6), the difference was also significant (p = 0.02, according to the same statistical test). These results are shown in the Figure 3.

Ninety days after the brain infarction diagnosis the patients were evaluated by the modified scale of Rankin (Figure 4). Patients with a score between 0 and 2 evolved satisfactorily, with fewer neurological sequelae and less functional disability. On the contrary, patients with scores above 3 had a worse neurological status, and greater functional disability. The highest rating on the Rankin scale is equivalent to the worst condition; in this evaluation the patients who died were assigned the value of 5 on the Rankin scale. The group of patients that received the therapy with 0.8 µg/kg body weight of EGF, and of 50 µg/kg body weight of GHRP6 showed a neurological improvement with respect to the group treated with the other combination (10 µg/kg body weight of EGF, and of 5 µg/kg body weight of GHRP6), and also with respect to the group treated with conventional therapy (Chi square test for proportions' comparisons). This difference is remarkable even in patients with the worst neurological status at the time of diagnosis (NIHSS> 15).

In addition to the clinical evaluation at 90 days after the brain infarction, serum galectin-3 levels were determined. The concentration of this lectin was measured by a commercially available ELISA immunoassay (R&D Systems, Minneapolis, United States). The three groups were compared, by means of the Kruskal Wallis test, followed by the Dunn test. A significant reduction of this biomarker was demonstrated in patients treated with the pharmaceutical combination of 0.8 µg/kg body weight of EGF, and of 50 µg/kg body weight of GHRP6, with respect to patients treated with conventional therapy, or with the other combination of EGF and GHRP6 (Figure 5).

The latter result, in addition to confirming the evidence from the proteomic study carried out in the preclinical evaluations (Example 1), shows that treatment with the pharmaceutical combination of 0.8 µg/kg body weight of EGF, and of 50 µg/kg body weight of GHRP6 decreases the galectin-3 serum levels. This is one of the lectins that activate microglia, through the toll-like receptor (namely, TLR4), and therefore contributes to the reduction and restrain of the brain inflammatory response, which is associated with the worst prognosis. This serum reduction of galectin-3 is related to a more favorable brain restoration induced by the pharmaceutical combination of the present invention.

In this clinical study, a time of inclusion of patients of up to 24 hours after the onset of neurological symptoms was established, in order to evaluate if the effect of this combination could be seen beyond the classical therapeutic window established for the interventions in patients with brain infarction (Mowla A, et al., J Neurol Sci 2017;376:102-5). Surprisingly, the group of patients treated with the pharmaceutical combination of 0.8 µg/kg body weight of EGF, and of 50 µg/kg body weight of GHRP6, did not show significant correlation between the starting time of treatment and the clinical outcome at 90 days, measured by the Rankin scale (Linear regression analysis and Sperman's r = -0.1, p =0.45) (Figure 6A). On the contrary, the group that received the other combination (10 µg/kg body weight of EGF, and of 5 µg/kg body weight of GHRP6), a positive and significant correlation was demonstrated between the delayed start treatment and the worst clinical outcome according to the Rankin scale (Linear regression analysis and Sperman's r = 0.36, p = 0.03) (Figure 6B). This result is very important because it means that the administration of the treatment at any time during the 24-hour period after the onset of symptoms, is associated with therapeutic benefit. Additionally, this result extends the eligibility criteria to patients in need for brain restoration, and contrasts with the results achieved by the neuroprotective therapeutic interventions that precede this invention.

Cognitive function frequently deteriorates in patients suffering from brain infarction. Threrefore cognitive function is one of the most important targets of the mechanisms of neuronal plasticity, and it is susceptible to be recovered when the brain restoration is successful. In the present study, a test for cognitive evaluation (cognitive evaluation of Montreal, MoCA) was applied at 2 and 6 months after the brain infarction episode, to patients treated with the pharmaceutical combination of 0.8 µg/kg body weight of EGF, and of 50 µg/kg body weight of GHRP6, to patientes who received treatment with the combination of 10 µg/kg body weight of EGF, and of 5 µg/kg body weight of GHRP6, and to the group that was treated with conventional therapy. We also included a control group of 30 patients affected by non-neurological diseases, paired by age and gender with the brain infarction patients of this study. The results of this trial indicated that patients treated with the pharmaceutical combination of 0.8 µg/kg body weight of EGF, and of 50 µg/kg body weight of GHRP6, after two months of said treatment, had a marked improvement in cognitive function as compared to patients who received conventional therapy, or therapy with the other combination (10 µg/kg body weight of EGF, and of 5 µg/kg body weight of GHRP6). At 6 months, this difference in cognitive function is not only maintained in the group treated with the combination of 0.8 µg/kg body weight of EGF, and of 50 µg/kg body weight of GHRP6 (statiscally significant), but, surprisingly, the cognitive function in this group was similar to that of patients with non-neurological diseases, (controls) (Figure 7).

### Example 3. Effect of the administration of the combination of EGF and the hexapeptide secretagogue in brain restoration in patients with Amyotrophic Lateral Sclerosis.

Forty patients with definite diagnosis of Amyotrophic Lateral Sclerosis were treated with EGF 0.6 µg/kg of body weight, and GHRP6 60 µg/kg of body weight, intravenously, every other day, during 6 months, together with Rilutek® (Riluzole). Previously, these patients were stratified according to the origin of the disease, spinal (n = 25) or bulbar (n = 15). Another group of 30 patients with definite diagnosis of Amyotrophic Lateral Sclerosis, also stratified according to origin, spinal (n = 21) or bulbar (n = 9), were treated only with Rilutek®.

Before the beginning and after the end of the trial, all patients were evaluated using the functional scale for Amyotrophic Lateral Sclerosis (ALSFRS-R). The ALSFRS-R scale is usually used for the neurological and functional evaluation of patients with said disease. This scale includes various functional items, such as those related to language, salivation, swallowing, the ability to write, handling eating utensils, dressing and sanitizing, turning in bed, climbing stairs, and breathing.

After 6 months of treatment, patients treated simultaneously with EGF 0.6 µg/kg of body weight, and of the GHRP6 60 µg/kg of body weight and Rilutek® showed some clinical improvement, as shown in Table 1. This improvement was found in patients with both spinal onset and bulbar onset, and consisted in decreased salivation, swallowing and breathing improvement and a slight improvement in dysarthria. None of the latter was observed in the group of patients treated only with Rilutek®.

**Table 1. Comparison of ALSFRS-R before and after treatment with the EGF-GHRP6 combination and Rilutek® or Rilutek® alone.**

| **Group** | **Origin of the symptoms of the disease** | **ALSFRS-R before treatment** | **ALSFRS-R after the treatment** |
|---|---|---|---|
| EGF 0,6 µg/kg y GHRP6 60 g/kg, + Rilutek® | spinal | 38,4±6,2 | **39,4±3,3** |
| | bulbar | 35,3±7,1 | **36,9±3,1** |
| Rilutek® | spinal | 38,7±4,4 | 38,2±3,34 |
| | bulbar | 35,1±6,2 | 33,1±8,32 |

### Example 4. Effect of the administration of the EGF-GHRP6 combination in brain restoration in newborns with severe brain hypoxia.

A group of newborns who, for various perinatal causes, had severe brain hypoxia received treatment, in a compassionate modality, with EGF 0.3 µg/kg of body weight, and of the GHRP6 30 µg/kg of body weight (n=9). The treatment was carried out for a week, and consisted in the parenteral administration of the EGF-GHRP6 combination, every 12 hours.

At the time of diagnosis, all newborns had electroencephalographic alterations of the slow wave type, and in the ultrasound examination there were signs of edema and echogenicity in periventricular areas. From the clinical point of view, there were signs of mild pulmonary hypertension, arterial hypotension, difficulty maintaining body temperature, hypoglycemia, and diuresis less than 1 mL per hour. In all cases seizures were recorded during the first 24 hours, and, in the majority, a weak suction reflex and miosis were observed.

Infants were followed during one year, with quarterly consultations. In the evaluation of the first trimester, 7 of the children treated with the indicated doses of EGF and GHRP6, had an electroencephalogram characterized by the presence of periodic multifocal discharges, but less frequent than those previously evaluated perinatally, and in all cases the babies reacted to intermittent light stimulation. The ultrasonographic examination showed that the brain periventricular cystic lesions were in clear regression. The physical examination, specifically the neurological evaluation, according to the Amiel-Tison scale, revealed a mild dysfunction of the central nervous system, consisting of slight disorders of muscle tone and reflexes, motor activity disorders and, in general, a delay in psychomotor development, with a mild visual and auditory deficit. At six months, 8 of the 9 children treated with the EGF-GHRP6 combination had a normal electroencephalogram. In the waking state rhythmicity was observed, and reactivity to intermittent luminous stimulation in the theta band. In sleep state there was hypnogogic hypersynchrony, with paroxysmal elements, but not epileptiform. The magnetic resonance images showed that the periventricular cystic lesions had disappeared, and the normal appearance of the basal nuclei was confirmed by computed tomography. In the neurological examination, there were neither disturbances in muscle tone and reflexes, nor in motor activity. A normal psychomotor development accoring to age was evidenced. Visual or auditory deficits were not detected. In general, the babies had a good response to the social environment.

At one year of age, the encephalogram pattern was normal in 8 of the 9 treated children, during the vigil a basic rhythm of posterior regions of 6-7 Hz, biphasic medium-high voltage potentials, and bilateral synchrony in occipital regions was evidenced to the blink. Reactivity to intermittent light stimulation was observed at low frequencies. The sleep recording did not show irregular REM activity, but symmetric and synchronous sleep spindles were evidenced. The treatment with the EGF-GHRP6 combination compasional at the beginning, showed that this pharmacological intervention in neonates with severe hypoxia was therapeutically efficient, because the evolution of these infants was much more favorable than that of the newborns with severe hypoxia treated only with conventional therapy, who showed a significant delay in neurological development according to age.

## Claims

1. A pharmaceutical combination for the restoration of brain damage comprising the epidermal growth factor (EGF) and the growth hormone secretagogue peptide GHRP6.

2. The combination according to claim 1 comprising 0.3 µg - 0.9 µg of EGF per kg of body weight and 30 µg - 80 µg of GHRP6 per kg of body weight.

3. The combination according to claim 2 wherein the EGF and the GHRP6 are administered sequentially or simultaneously by parenteral route.

4. The combination according to claim 3 wherein the parenteral administration is carried out intravenously, intrathecally, intracerebroventricularly, intra-cistern magna or intranasally.

5. The combination according to claim 4 wherein intrathecal administration is performed by a lumbar puncture and/or a device designed for administration by said route.

6. The combination according to claim 1 consisting of a kit comprising: a) a container comprising EGF and pharmaceutically acceptable excipients and b) a container comprising GHRP6 and pharmaceutically acceptable excipients.

7. The combination according to claim 6 wherein the container comprising EGF comprises between 5 µg and 80 µg of EGF, and the container comprising GHRP6 comprises between 0.5 mg and 6 mg of GHRP6.

8. Use of epidermal growth factor (EGF) and the growth hormone secretagogue peptide GHRP6 for the manufacture of a pharmaceutical combination for the restoration of brain damage.

9. The use according to claim 8 wherein the combination is used in the restoration of brain damage after brain infarction.

10. The use according to claim 9, wherein the first administration of the combination is carried out between the initial moment of diagnosis of brain infarction and 24 hours after diagnosis.

11. The use according to claim 8 wherein the combination comprises 0.3 µg - 0.9 µg of EGF per kg of body weight and 30 µg - 80 µg of GHRP6 per kg of body weight.

12. A method of restoring brain damage wherein a therapeutically effective amount of a pharmaceutical combination comprising epidermal growth factor (EGF) and growth hormone secretagogue peptide GHRP6 is administered to a patient in need thereof.

13. The method according to claim 12 wherein the combination comprises 0.3 µg - 0.9 µg of EGF per kg of body weight and 30 µg - 80 µg of GHRP6 per kg of body weight.

14. The method according to claim 12 wherein the brain damage is of an ischemic, toxic, surgical, traumatic nature, by proteinopathies, genetic disorders, or other causes of permanent neuronal damage.

15. The method according to claim 14 wherein the brain damage is caused by brain infarction, severe brain hypoxia in newborns or Amyotrophic Lateral Sclerosis.

16. The method according to claim 12 wherein the combination is administered in the treatment of motor neuron diseases of progressive and torpid course to improve muscle strength, swallowing reflex and to reduce dysarthria.

17. The method according to claim 12 wherein the combination is administered for the preservation of complex functions such as memory, learning ability, recovery of motor, sensory and cognitive abilities in humans.
